# EUROPEAN PATENT SPECIFICATION

(11) **EP 4 333 831 B1**
(45) Date of publication and mention of the grant of the patent: **14.08.2024**
(21) Application number: 23715892.8
(22) Date of filing: 14.04.2023
(51) Int. Cl.: A61K 31/216, A61K 31/40, A61K 31/439, A61P 17/00, A61K 9/00, A61K 9/06, A61K 47/10

(54) **TOPICAL FORMULATION OF AN ANTICHOLINERGIC COMPOUND FOR TREATING SEVERE HYPERHIDROSIS AND EXCESSIVE SWEATING**
TOPISCHE FORMULIERUNG EINER ANTICHOLINERGEN VERBINDUNG ZUR BEHANDLUNG VON SCHWERER HYPERHIDROSE UND ÜBERMÄSSIGEM SCHWITZEN
FORMULATION TOPIQUE D'UN COMPOSÉ ANTICHOLINERGIQUE POUR LE TRAITEMENT DE L'HYPERHIDROSE SÉVÈRE ET DE LA TRANSPIRATION EXCESSIVE

(30) Priority: 14.04.2022 EP 22168549; 10.06.2022 EP 22178325
(43) Date of publication of application: 13.03.2024
(73) Proprietor: Dr. August Wolff GmbH & Co. KG Arzneimittel, 33611 Bielefeld (DE)
(72) Inventor: DR. MASUR, Clarissa, 33611 Bielefeld (DE); DR. SCHULZE ZUR WIESCHE, Erik, 33611 Bielefeld (DE); DR. NEUHAUS, Bernhard, 33611 Bielefeld (DE)
(74) Representative: Ter Meer Steinmeister & Partner
(86) International application number: PCT/EP2023/059763
(87) International publication number: WO 2023/198876

(56) References cited:
- US-A1- 2021 290 536
- ABELS C. ET AL: "A glycopyrronium bromide 1% cream for topical treatment of primary axillary hyperhidrosis: efficacy and safety results from a phase IIIa randomized controlled trial*", vol. 185, no. 2, 2 March 2021 (2021-03-02), Hoboken, USA, pages 315 - 322, XP055958742, ISSN: 0007-0963, Retrieved from the Internet <URL:https://onlinelibrary.wiley.com/doi/full-xml/10.1111/bjd.19810> DOI: 10.1111/bjd.19810

## Description

The present invention relates to a topical formulation comprising at least one anticholinergic compound for use in treating patients suffering from severe hyperhidrosis, wherein administration of the formulation to the patient comprises: a) an initial phase with a topical administration of a predetermined amount of the formulation 5 to 7 times per week for a total time period of less than six months; followed by b) a maintenance phase with a topical administration of the same amount of the formulation 1 to less than 5 times per week, wherein the anticholinergic compound is a glycopyrronium (GP) salt that is contained in an amount of 0.05 to 5 wt.%, based on the weight of the total formulation.

Hyperhidrosis is a disorder of excessive sweating beyond what is required to maintain normal thermal regulation of the human body. Thus, hyperhidrosis is an extremely inconvenient condition negatively affecting daily life of a person suffering therefrom. Hyperhidrosis can be divided into focal and general hyperhidrosis. The focal is bilaterally symmetrical: hands, feet, axillae or groins. Focal hyperhidrosis from the face/head does occur but is often part of the general form. Generalized sweating usually involves both the head and trunk and in severe cases also extremities and groins/glutes. Whereas the majority of patients affected have the primary form which is hereditary, there is also a secondary form, often related to an underlying disease.

The Hyperhidrosis Disease Severity Scale (HDSS; for details see e.g. Haider A, Solish N. Focal hyperhidrosis: diagnosis and management. CMAJ 2005;172:69-75; Solish N, Bertucci V, Dansereau A, Hong HC, Lynde C, Lupin M, et al. A comprehensive approach to the recognition, diagnosis, and severity-based treatment of focal hyperhidrosis: recommendations of the Canadian Hyperhidrosis Advisory Committee. Dermatol Surg 2007;33(8):908-23) is a 4-point scale designed to assess the severity of hyperhidrosis in everyday clinical practice and effectiveness of hyperhidrosis treatment. It is a disease-specific diagnostic tool that provides a highly reliable qualitative measure of the severity of the patient's condition based on how it affects daily activities. A score of 3 or 4 indicates severe hyperhidrosis. A score of 1 and 2 indicates mild and moderate hyperhidrosis, respectively. An improvement of 1 HDSS score corresponds to 50% and an improvement of 2 HDSS scores to 80% reduction in sweat production.

A variety of treatments have been proposed for treating excessive sweating/hyperhidrosis which include aluminum containing antiperspirants, surgical removal of sweat glands and systemic or local treatment with anticholinergic compounds. Any of these treatments, however, entail serious disadvantages. Aluminum containing antiperspirants, in particular upon continuous use, stain or discolor the textiles coming into contact with the antiperspirant. In addition, the increasing amount of aluminum in waste water recently has also been increasing environmental concerns. On the other hand, removal of the sweat glands means surgery that may be accompanied by at least inconvenient or even serious side effects.

As an alternative to the treatments mentioned above, anticholinergic compounds such as glycopyrronium salts (also named glycopyrrolates; GP salts) have been proposed for the treatment of excessive sweating and hyperhidrosis. Abels C. et al: "A glycopyrronium bromide 1% cream for topical treatment of primary axillary hyperhidrosis: efficacy and safety results from a phase IIIa randomized controlled trial" discloses the use of glycopyrronium bromide in the treatment of severe hyperhidrosis. According to this document, the topical formulation of glycopyrronium needs to be applied daily in order to be effective in the treatment of patients with severe hyperhidrosis. Further, Glaser Dee Anna ET AL:"A-44-Week Open-Label Study Evaluating Safety and Efficacy of Topical Glycopyrronium Tosylate in Patients with Primary Axillary Hyperhidrosis" discloses the safe and efficacious once daily treatment of severe hyperhidrosis with glycopyrronium tosylate. WO 2006/069998 discloses the use of glycopyrronium bromide together with a large variety of other active ingredients for the treatment of excessive sweating. WO 2014/134510 relates to a specific glycopyrronium tosylate salt for the treatment of excessive sweating and hyperhidrosis. WO 2020/020879 discloses the treatment of patients suffering from only mild/moderate hyperhidrosis (namely with a score of 1 or 2 according to the HDSS). This document does not contain any data that would support the transfer from mild/moderate hyperhidrosis to severe hyperhidrosis.

However, all of these known compositions basically suffer from at least one insufficiency selected from instability of the composition, inadequate cosmetic acceptance and skin care characteristics, systemic exposure, systemic side effects and/or limited efficacy in the treatment of severe hyperhidrosis and/or excessive sweating.

In view thereof there is still need in the prior art for improved methods of treating severe hyperhidrosis and/or excessive sweating.

Therefore, the object underlying the present invention is the provision of an improved treatment of severe hyperhidrosis and/or excessive sweating which require only a minimum amount of active ingredient while at the same time leading to a maximum efficacy.

This object is achieved by a topical formulation, a pharmaceutical composition comprising the same, and the use of the formulation/pharmaceutical composition in treating severe hyperhidrosis, as defined in the claims attached. In particular, it was surprisingly found out that by dividing administration (i.e. the administration regimen) of a topical formulation to the patient suffering from severe hyperhidrosis particularly into a specific initial phase of administration and a specific maintenance phase of administration, only a minimum amount of active ingredient is required while nevertheless a maximum efficacy is provided. This is particularly important in view of the fact that (primary) hyperhidrosis is a chronic disease that regularly requires a continuous treatment. At the same time it was surprisingly found that local and systemic side effects (such as skin irritation or even inflammation at application site, application site pain, dry mouth, dry skin, dry eye, blurred vision, mydriasis, constipation, urinary hesitation, urinary retention, and nasal dryness, known from e.g. glycopyrronium salts containing compositions) are significantly reduced while the patient's compliance is even improved. In other words, according to the present invention it was surprisingly found that, even with the administration of the reduced amount of active ingredient (i.e. glycopyrronium salts) in accordance with the administration regimen of the present invention, the therapeutic window of the treatment of severe hyperhidrosis can successfully be fulfilled/achieved, in particular in long-term. The administration regimen of the invention also allows for an improved patient compliance upon long-term application, together with an improved economic efficiency. In the prior art it was not possible to sufficiently fulfill these properties all together at the same time. Moreover, there was no motivation existing in the prior art to reduce the treatment frequency of the glycopyrronium salts to less than once daily with the expectation of a still sufficient efficacy in the treatment of severe hyperhidrosis. In fact, according to the Glaser publication mentioned above it was considered necessary to apply glycopyrrolate once daily over a period of 44 weeks to maintain a plateau efficacy level. Thus, the skilled person would not have reduced administration frequency. Quite in contrast, the skilled person would have expected a limited and insufficient efficacy in the treatment of severe hyperhidrosis when reducing the administration regimen to below once daily.

According to the present invention the administration regimen is divided into several distinct phases of administration. These phases basically differ in their frequency of topical application of the formulation to the patient's skin (application or administration per time period) and preferably comprise, even more preferably consist of, an initial phase of administration (also defined herein as initiation phase) and a maintenance phase of administration (also defined herein as continuation or continuous phase). The administration starts with the initial phase, followed by the maintenance phase. The maintenance phase preferably immediately (directly) follows the initial phase. Alternatively, there may be a "transition phase" during which the administration regime of the initial phase is step-wise reduced to achieve the administration regime of the maintenance phase. This helps the patients to carefully adapt the administration according to the invention while maximizing compliance and efficacy. The several phases are also named herein as the respective "time phases" or "time periods" (such as the initial time phase/time period of administration and the maintenance time phase/time period of administration). While not being limited thereto, administration of the invention preferably comprises only one initial phase and one maintenance phase over the total treating time of the patient suffering from severe hyperhidrosis. However, it is also included within the invention to return from the administration frequency of the maintenance phase back to the administration frequency of the initial phase and again back to the administration frequency of the maintenance phase, in case efficacy considerations of the treatment require. In other words, the present invention also includes administration with repeatedly interchanging "initial phases" and "maintenance phases" over the total treating time of the patient suffering from severe hyperhidrosis.

According to the invention the amount of the topical formulation (and consequently also the amount of the active ingredient, namely the anticholinergic compound being a GP salt) applied per administration remains the same, both in the initial phase and the maintenance phase. The amount is also defined as the "predetermined amount" herein. In this respect the terms "amount" of the topical formulation, "dose" of the topical formulation and "dosage" of the topical formulation are considered identical and used interchangeably. It will be understood by the skilled person that the respective amount also depends on the identity of the anticholinergic compound applied.

Administration of the topical formulation (also defined herein as administration frequency or application frequency) during the initial phase is 5 to 7 times per week (such as 5, 6, or 7 times per week, e.g. uniformly/evenly distributed or arbitrarily distributed over the week, most preferably uniformly/evenly distributed over the week, namely not more than once daily). The total time period (also named duration) of the initial phase is less than six months, preferably two weeks to 4 months, more preferably 3 weeks to 2 months, even more preferably 4-6 weeks, most preferably (around) 4 weeks/one month.

Administration of the topical formulation (also defined herein as administration frequency or application frequency) during the maintenance phase is from 1 to less than 5 times per week, more preferably 2 to 4 times per week, most preferably 2 to 3 times per week, and particularly preferably 2 times per week. Administration within these periods is either randomly distributed or uniformly distributed, preferably uniformly distributed for compliance reasons. Administration frequency depends on the treatment effect on severe hyperhidrosis and can be adapted accordingly. The total time period (also named duration) of the maintenance phase is not specifically limited but depends on the duration an individual suffers from hyperhidrosis. A minimum duration is several weeks to several years (such as from 4 to 8 weeks to 1.5 to 2 years, e.g. 3 to 8 months). The maximum duration is not particularly limited and ranges from several months to several years. Most preferably the administration according to the invention is a continuous administration over time.

According to the invention administration (preferably both within the initial phase and the maintenance phase) the topical formulation is applied in the evening such as prior to bedtime.

A particularly preferred administration regimen of the topical formulation according to the invention is once daily of a predetermined amount for 4 weeks (initial phase), immediately followed (i.e. starting with week 5) by administration of the same amount 2 to 3 times per week (maintenance phase). Alternatively, there is a "transition phase" between the initial phase and the maintenance phase of 1 to 12 weeks, preferably of 2 to 10 weeks, more preferably 3 to 8 weeks (such as 4, 5, 6, or 7 weeks). During this "transition phase" the administration frequency is step-wise reduced (basically following the patient's efficacy and compliance considerations). A particularly preferred reduction is a homogenous ("smooth" and/or "continuous") reduction of administration frequency over the time periods as indicated above. The maintenance phase in both cases is then continued for the rest of the hyperhidrosis treatment period.

According to the present invention it was surprisingly found that it is possible to reduce the administration frequency of a formulation comprising an anticholinergic compound after a defined initial phase of administration and to continue with the reduced administration frequency with the maintenance phase until the end of the treatment period without losing efficacy. Quite in contrast, it was found out that the treatment of severe hyperhidrosis and/or excessive sweating was highly effective following this administration regimen. This was unexpected because due to the reduced administration frequency the total amount of anticholinergic compound applied was significantly reduced as compared to the prior art treatment with a constant (and constantly high) administration frequency. Importantly the same formulation with the same dose per application was used in the initial phase and in the maintenance phase of administration. Only the frequency of administration was changed. High efficacy of the inventive treatment is proven by a reduction and maintenance of the HDSS score by a value of 1 or 2, preferably by 2 (i.e. from HDSS score 3 or 4 to HDSS score 1 or 2). At the same time it was surprisingly found that local and systemic side effects are significantly reduced while a good patient's compliance is achieved. It was particularly unexpected to keep the high efficacy with a reduced administration regimen even with patients suffering from severe hyperhidrosis and/or excessive sweating. In conclusion, the present invention unexpectedly leads to an improved overall performance of the treatment of severe hyperhidrosis and/or excessive sweating.

The anticholinergic compounds used according to the present invention are glycopyrronium (GP) salts. GP salts chemically mean (2-cyclopentyl-2-hydroxy-2-phenylacetyl)oxy]-1,1-dimethylpyrrolidinium salts. The salts that may be used according to the present invention are not particularly limited, and actually any salts may be used. These include the chloride, bromide, fluoride, iodide, nitrate, sulfate, sulfonate, and phosphate salts of glycopyrronium (GP). Also suitable are the acetate, propionate, glycolate, pyruvate, oxalate, succinate, fumarate, tartrate, citrate, benzoate, methanesulfonate, 4-methylbenzenesulfonate (tosylate), and salicylate salts of glycopyrronium (GP). Particularly preferably the salts are selected from the group consisting of the bromide, the acetate, and the tosylate salts of glycopyrronium (GP)The emulsion of the invention may contain one, two, three or more different salts of glycopyrronium (GP). However, preferably it contains only one GP salt, such as the bromide, the acetate, and the tosylate salts of glycopyrronium (GP). Most preferably the anticholinergic compound is GP bromide.

The glycopyrronium (GP) salts of the present invention include the racemic mixtures thereof. For example, the GP salt according to the present invention may be a racemic mixture of the (3*R*)-3-[(2*S*)-(2-cyclopentyl-2-hydroxy-2-phenylacetyl)oxy]-1,1-dimethylpyrrolidinium and (3*S*)-3-[(2*R*)-(2-cyclopentyl-2-hydroxy-2-phenylacetyl)oxy]-1,1-dimethylpyrrolidinium salts. Preferably a racemic mixture of (3*R*)-3-[(2*S*)-(2-cyclopentyl-2-hydroxy-2-phenylacetyl)oxy]-1,1-dimethylpyrrolidinium bromide and (3*S*)-3-[(2*R*)-(2-cyclopentyl-2-hydroxy-2-phenylacetyl)oxy]-1,1-dimethylpyrrolidinium bromide or a racemic mixture of (3*R*)-3-[(2*S*)-(2-cyclopentyl-2-hydroxy-2-phenylacetyl)oxy]-1,1-dimethylpyrrolidinium tosylate and (3*S*)-3-[(2*R*)-(2-cyclopentyl-2-hydroxy-2-phenylacetyl)oxy]-1,1-dimethylpyrrolidinium tosylate is used.

The formulation according to the present invention contains the GP salt such as the bromide (GPB), in an amount of 0.05 to 5 wt.%, preferably of 0.1 to 3 wt.%, more preferably of 0.2 to 2 wt.%, and even more preferably of 0.5 to 1.5 wt.%, based on the weight of the total formulation. When the GP salt such as the bromide (GPB), is contained in an amount within these ranges a high efficacy in reducing excessive sweating is achieved in patients suffering from severe hyperhidrosis rated as 3 or 4, preferably as 4, in the Hyperhidrosis Disease Severity Scale (HDSS), while at the same time potential undesirable side effects of GP salts can be avoided. Furthermore, surprisingly a stable formulation can be obtained even with high amounts of GP salt (namely even with 2 to 5 wt.% and in particular with 2 to 3 wt.%). Overall, an amount of about 1.0 wt.% GP salt (in particular GPB or GP tosylate, most preferably GPB) is most preferred especially from a cost, efficacy and side effect (safety) perspective.

The topical formulation containing GPB (e.g. as a 0.5 to 2.0 wt.%, most preferred as a 1.0 wt.% formulation, based on the weight of the total formulation) is preferably administered in an amount within a range of from 200 to 800 mg, preferably from 400 to 600 mg, most preferably 500 to 550 mg (such as 540 mg) per application. In a most preferred embodiment the formulation is administered in an amount of 540 mg per application per axilla.

The formulation of the present invention may be in any form that allows the topical application onto the skin of a mammal. This includes, without being limited thereto, an emulsion (such as a W/O, O/W, W/O/W, O/W/O emulsion), a gel, a solution, and a mixed form of a gel and an emulsion (emulgel). Preferably the formulation is an emulsion. According to the present invention a topical emulsion may be either a water-in-oil emulsion (also named W/O emulsion) or an oil-in-water emulsion (also named O/W emulsion). In a preferred embodiment the topical emulsion of the present invention is an oil-in-water emulsion (O/W emulsion) comprising one or more anticholinergic compound(s) as active ingredient, wherein the emulsion contains a disperse (inner) oil phase, a continuous (outer) water phase containing the anticholinergic compound, and an emulsifier. Namely, the anticholinergic compound due to its hydrophilic property and its water solubility is mainly contained in the water phase itself and in particular at the interface of the water and oil phases of the emulsion.

The oil phase of the topical emulsion of the present invention, and preferably of the O/W emulsion, may be a typical oil phase of emulsions known in the prior art. Preferred as the main (basic) oil ingredient of the oil phase, however, are linear or branched long chain fatty alcohols having 6 to 36 carbon atoms, preferably having 6 to 22 carbon atoms. More preferably according to the present invention the oil phase contains octyldodecanol because of its skin smoothening and moisturizing effects and stability against oxidation even under acidic conditions (e.g. pH 4). Octyldodecanol further improves the permeation behaviour of the emulsion of the present invention. Thus, the oil phase containing octyldodecanol is preferred because it provides improved skin care (e.g. smoothening and moisturizing) effects corresponding to an improved cosmetic acceptance of the patient or user.

The water phase comprises mainly water as solvent and the anticholinergic compound as active ingredient for treating hyperhidrosis. The water phase may also comprise water soluble/miscible compounds (e.g. lower alcohols such as ethanol or isopropanol), preservatives (such as benzyl alcohol or phenoxyethanol), pH adjusting agents or buffers (such as citric acid/citrate), as well as moisturizing agents (such as propylene glycol) and other typical hydrophilic pharmaceutical/cosmetic excipients. However, it is preferred not to incorporate glycerol itself into the emulsion of the invention because glycerol has been shown to provide a detrimental effect on stability of the emulsion.

The topical emulsions, and preferably the O/W emulsions, of the present invention contain a smaller oil phase as compared to the water phase. Preferably the oil phase ranges from 10 to 25 %, and the water phase ranges from 65 to 80 wt.% of the total weight of the emulsion. Despite of the relatively small ratio of the oil phase the emulsion of the invention surprisingly provides both for better stability (no phase separation) and for improved skin care characteristics, as already mentioned above. At the same time it was unexpected that the release rate of anticholinergic compounds from the larger water phase was increased. Actually, the opposite was expected, namely a decreased release rate due to the larger water phase and the high water solubility and hydrophilic properties of the anticholinergic compounds. This allows for further reducing the amount of the anticholinergic compound leading to a better side effect profile without reducing efficacy.

The emulsifier of the topical emulsion may be any emulsifier suitable to provide for a W/O or O/W emulsion. Preferably the emulsifier provides for an O/W emulsion.

A particularly preferred emulsifier according to the present invention is an emulsifier system that contains at least three different components, namely at least one macrogol glycerol fatty acid ester, at least one glycerol fatty acid ester, and at least one fatty alcohol. In the present application the term "macrogol", as common in the pharmaceutical field, is used as a synonym for "polyethylene glycol (PEG)". This is also supported by the skilled person's common general knowledge. Preferably, the emulsifier system comprises a combination of a macrogol glycerol fatty acid ester, a glycerol fatty acid ester, and a fatty alcohol. As the fatty alcohol it is preferred to use a mixture of two fatty alcohols. By using the specific emulsifier system in the topical (O/W) emulsion of the present invention the above mentioned effects (i.e. efficacy with patients suffering from severe hyperhidrosis rated as 3 or 4 in the Hyperhidrosis Disease Severity Scale (HDSS), improved side effect profile, improved compliance and cosmetic acceptance/skin care characteristics also including improved stability, increased release rate) can be achieved.

More preferably with respect to the above described effects, the emulsifier system comprises a combination of macrogol glycerol monostearate, glycerol monostearate, and a mixture of cetyl alcohol and stearyl alcohol (e.g. with minimum 40.0 wt.% stearyl alcohol). Even more preferably, the emulsifier system comprises a combination of macrogol 20 glycerol monostearate, glycerol monostearate 40-55, and cetostearyl alcohol. Cetostearyl alcohol is a mixture of fatty acid alcohols, in particular a mixture of cetyl and stearyl alcohols. Most preferably the emulsifier system (essentially) consists of these indicated combinations.

The topical (O/W) emulsion according to the present invention may contain the respective components of the emulsifier system in the following amounts: 4.0 to 10.0 wt.%, preferably of 5.0 to 9.0 wt.%, more preferably of 6.0 to 8.0 wt.%, and even more preferably of 6.5 to 7.5 wt.% of the macrogol glycerol fatty acid ester; 1.5 to 7.5 wt.%, preferably of 2.5 to 6.5 wt.%, more preferably of 3.5 to 5.5 wt.%, and even more preferably of 4.0 to 5.0 wt.% of glycerol fatty acid ester; 5.0 to 11.0 wt.%, preferably of 6.0 to 10.0 wt.%, more preferably of 7.0 to 9.0 wt.%, and even more preferably of 7.5 to 8.5 wt.% of fatty alcohol, based on the weight of the total emulsion, respectively. By applying these preferred ranges the stability, cosmetic acceptance and efficacy is further improved.

The emulsifier system of the topical (W/O) emulsion according to the present invention may contain the respective components in the following ratios: 21 to 51 wt.%, preferably of 26 to 46 wt.%, more preferably of 31 to 41 wt.%, and even more preferably of 33 to 38 wt.% of macrogol glycerol fatty acid ester; 8 to 38 wt.%, preferably of 13 to 33 wt.%, more preferably of 18 to 28 wt.%, and even more preferably of 21 to 26 wt.% of glycerol fatty acid ester; 26 to 56 wt.%, preferably of 31 to 51 wt.%, more preferably of 36 to 46 wt.%, and even more preferably of 38 to 44 wt.% of fatty alcohol (or fatty alcohol mixture), based on the weight of the emulsifier system, respectively. By applying these preferred ratios the stability, cosmetic acceptance and efficacy is further improved.

The topical formulation of the present invention can be used as a pharmaceutical composition/medicament or as a cosmetic composition, depending on the indication to be treated and the respective regulatory requirements.

The formulations/compositions in accordance with the present invention may contain cosmetically and pharmaceutically/dermatologically acceptable excipients known to the skilled person. These include, next to the ones already mentioned above, for example further solvents such as organic solvents, gelling agents, buffers, detergents, emulsifiers, solubilizers, humectants, fillers, bioadhesives, emollients, preservatives, bactericides, surfactants, perfumes, thickeners, softening agents, moisturizing agents, oils, fats, waxes, water, alcohols, polyols, polymers, foam stabilizers, foaming agents, anti-foaming agents, or other suitable components of a pharmaceutical or cosmetic preparation.

Examples for bactericides are organic acids like formic acid, sorbic acid and benzoic acid. In addition esters of p-hydroxybenzoic acid, formaldehyde-releasing agents like DMDM hydantoin, imidazolidinylurea or methyl chloroisothiazolinone, methylisothiazolinone, dibromodicyanobutane, iodopropynyl butylcarbamte, phenoxyethanol or benzyl alcohol can be used as bactericides.

Further, pharmaceutically/dermatologically acceptable excipients according to the present invention may be inorganic or organic substances for topical administration. The pH value of the formulation can be stabilized using buffer systems consisting of polyacids and their salts. Examples for such polyacids are citric acid, tartaric acid and malic acid.

In the present invention, the formulation is preferably a dermatological composition suitable to be applied topically on the skin of a mammal. The form of the composition is not particularly limited. In preferred embodiments, the compositions are emulsions preferably in the form of lotions, creams, sprays, shampoos, foams, or saturated pads, wipes or cloth. Preferably they can be applied using a dispenser. A particularly suitable dispenser is disclosed in WO 2020/208063 that is designed to dispense fixed and constant dosages of formulation (per pump actuation).

Patients to be treated according to the present invention are defined as patients suffering from severe hyperhidrosis rated as 3 or 4, preferably as 4, in the Hyperhidrosis Disease Severity Scale (HDSS). Next to HDSS there are alternative definitions existing in the prior art to define severe hyperhidrosis. These are included in the present invention as well. "Severe Hyperhidrosis" herein is intended to comprise all clinical disorders/indications associated with excessive sweating, in particular primary axillary hyperhidrosis. These hyperhidrosis disorders may include severe forms of primary and secondary hyperhidrosis, gustatory sweating associated with Frey's syndrome, gustatory sweating associated with diabetic autonomic neuropathy, and excessive sweating in general. In particular, the following indications can be treated according to the present invention and are associated with dermatology, e.g. eccrine nevus, idiopathic unilateral focal hyperhidrosis, vascular deformities, pretibial myxedema; associated with gynaecology, e.g. postmenopausal hyperhidrosis; iatrogenic, such as associated with medicines, namely methadone or other opiates, cholinergics such as galantamine, SSRIs; associated with infection, e.g. brucellosis, HIV, chronic malaria, TBC, endocarditis; associated with surgery e.g. compensatory hyperhidrosis after sympathectomy; associated with medicines, such diabetes (hyperhidrosis due to neuropathy or hypoglycaemia), endocrine diseases (acromegaly, pheochromocytoma, hyperthyroidism, hypogonadism, insulinoma, heart failure, obesity); associated with neurology, e.g. central or peripheral lesion, Harlequin syndrome, Horner's syndrome, compensatory hyperhidrosis, Ross syndrome, Parkinson's disease, polyneuropathies; associated with oncology, such as carcinoid, lymphoma, with several malignancies; associated with orthopaedics, e.g. hyperhidrosis from amputation stump; associated with psychiatry, e.g. anxiety disorder, psychotropic drugs, social phobia.

The formulation of the present invention is applied topically on the skin of a mammal. Typical application sites include the face (e.g. forehead, chin, neck and scalp), armpit (axilla), underarms, palms of the hands, soles of the feet, backs of the knees, trunk, and groin. A particularly preferable application site according to the invention is the armpit (axilla).

The following examples show preferred embodiments of the invention.

### Examples

The following examples have been prepared by combining the indicated ingredients using a conventional mixer (e.g. Becomix RW 320). All ingredients are commercially available. Macrogol 20 glycerol monostearate is also known as polyethylene glycol (PEG)-20 glyceryl stearate. Glycerol monostearate 40-55 is also known as glycerol monostearate 40-55% (Type II). Cetostearyl alcohol is a mixture of cetyl and stearyl alcohols.

### Preparation Example 1:

| **Position** | **Substance** | **g/100 g** |
|---|---|---|
| **1** | Macrogol 20 glycerol monostearate | 7.00 |
| **2** | Glycerol monostearate 40-55 | 4.50 |
| **3** | Cetostearyl alcohol | 8.00 |
| **4** | Octyldodecanol | 8.00 |
| **5** | Benzyl alcohol | 1.00 |
| **6** | Glycopyrronium bromide | 1.00 |
| **7** | Citric acid, anhydrous | 0.32 |
| **8** | Sodium citrate | 0.30 |
| **9** | Propylene glycol | 3.00 |
| **10** | Purified water | 66.88 |

### Preparation Example 2:

| **Position** | **Substance** | **g/100 g** |
|---|---|---|
| **1** | Macrogol 20 glycerol monostearate | 7.00 |
| **2** | Glycerol monostearate 40-55 | 4.50 |
| **3** | Cetostearyl alcohol | 8.00 |
| **4** | Octyldodecanol | 8.00 |
| **5** | Benzyl alcohol | 1.00 |
| **6** | Glycopyrronium bromide | 0.50 |
| **7** | Citric acid, anhydrous | 0.32 |
| **8** | Sodium citrate | 0.30 |
| **9** | Propylene glycol | 3.00 |
| **10** | Purified water | 67.38 |

### Preparation Example 3:

| **Position** | **Substance** | **g/100 g** |
|---|---|---|
| **1** | Macrogol 20 glycerol monostearate | 7.00 |
| **2** | Glycerol monostearate 40-55 | 4.50 |
| **3** | Cetostearyl alcohol | 8.00 |
| **4** | Octyldodecanol | 8.00 |
| **5** | Benzyl alcohol | 1.00 |
| **6** | Glycopyrronium bromide | 2.00 |
| **7** | Citric acid, anhydrous | 0.32 |
| **8** | Sodium citrate | 0.30 |
| **9** | Propylene glycol | 3.00 |
| **10** | Purified water | 65.88 |

All Preparation Examples exhibited a good stability of the O/W emulsions over 36 months during stability testing (stability of emulsion in terms of phase separation and in terms of stability of active pharmaceutical ingredient (API)).

### Example - Treatment of Severe Hyperhidrosis in Patients rated as 3 to 4 in the Hyperhidrosis Disease Severity Scale (HDSS)

Formulations according to the present invention were tested in a clinical trial assessing local and systemic tolerability and efficacy of glycopyrronium bromide (GPB) in a topical formulation in a placebo controlled, double blind study in subjects with severe axillary hyperhidrosis. 518 patients with primary axillary hyperhidrosis were enrolled in the study. According to the present invention only patients suffering from severe hyperhidrosis rated as 3 or 4 in the Hyperhidrosis Disease Severity Scale (HDSS) and at least 50 mg of sweat production over a period of 5 minutes were evaluated.

The patients received the 1%-GPB-containing cream of Preparation Example 1. During the first 4 weeks (initial phase) the patients applied a predetermined dose of the cream (540 mg cream, corresponding to 4.4 mg glycopyrronium) per axillae once daily. Following the initial phase the frequency of application was reduced to ultimately (in average) 3 times per week (maintenance phase). Over a period a 72 weeks (including initial phase and maintenance phase) the treatment success was maintained or even slightly improved with the lower frequency of product administration of the maintenance phase.

Treatment success is shown by means of reduction in HDSS and reduction of absolute sweat production assessed by gravimetric measurements. However, other parameters to define success of treatment are also possible, examples include but are not limited to other quality of life measurement tools such as DLQI, HidroQoL, Skindex-16 etc., or other means of objective sweat measurement such as minor tests and size of sweat stains on the clothes etc.

The HDSS questionnaire (Haider and Solish 2005; Solish et al. 2007) was completed at Screening, Day 1, after week 4, week 8, week 12, week 28, week 52 and week 72.

Patients were asked to select the statement that best reflected their experience with axillary Sweating according to below shown question and possible answers. A score of 3 or 4 indicated severe hyperhidrosis. A score of 1 or 2 indicated mild or moderate hyperhidrosis.

| Question: "How would you rate the severity of your hyperhidrosis?" | |
|---|---|
| Answer | Score |
| My underarm sweating is never noticeable and never interferes with my daily activities | 1 |
| My underarm sweating is tolerable but sometimes interferes with my daily activities | 2 |
| May underarm sweating is barely tolerable and frequently interferes with my daily activities | 3 |
| My underam sweating is intolerable and always interferes with my daily activities | 4 |

Gravimetric measurements of total sweat production were performed at Screening, Day 1, after week 4 and after week 12. Total axillar sweat during a defined time period was collected using a standardized filter paper and subsequently the amount of sweat was determined by gravimetry. The results are shown in Tables 1-3 below.

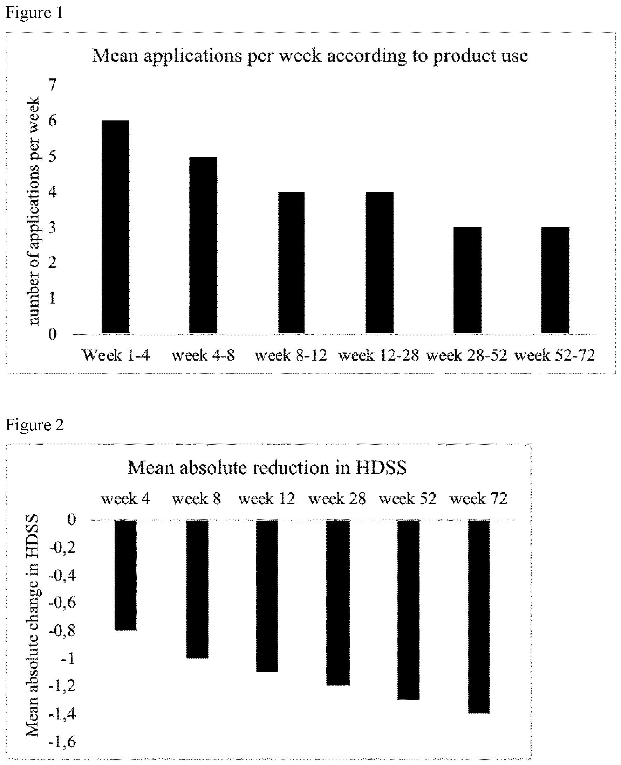

**Table 1: Mean number of applications per week calculated from the amount of product that was used during the indicated time periods (see also Figure 1).**

| | **Mean number of applications per week (n)** |
|---|---|
| week 1-4 | 6 |
| week 4-8 | 5 |
| week 8-12 | 4 |
| week 12-28 | 4 |
| week 28-52 | 3 |
| week 52-72 | 3 |

**Table 2: Mean change of HDSS score from baseline after indicated treatment periods (see also Figure 2 showing mean absolute change in HDSS score after 4, 8, 12, 28, 52 and 72 weeks of treatment comprising initial phase of treatment and maintenance phase of treatment. Change of HDSS score is statistically significant compared to Baseline at measurement points (p < 0.0001))**

| | **Mean change of HDSS from Baseline** |
|---|---|
| week 4 | -0,8 |
| week 8 | -1 |
| week 12 | -1,1 |
| week 28 | -1,2 |
| week 52 | -1,3 |
| week 72 | -1,4 |

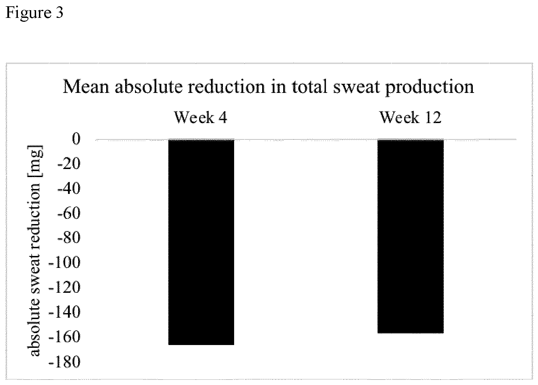

**Table 3: Absolute reduction of total axillary sweat production from baseline after indicated treatment periods (see also Figure 3 showing mean absolute reduction in total sweat production measured by gravimetry. During or after the initial phase sweat production is reduced by a mean of 166.46 mg compared to Baseline values. During the maintenance phase the reduction in sweat production is maintained with a mean value of 156.67 mg compared to Baseline values despite reduction in frequency of treatment. Reduction of total sweat production is statistically significant at all measurement points (p < 0.0001))**

| | **Mean absolute reduction in total sweat production [mg]** |
|---|---|
| week 4 | -166,46 |
| week 12 | -156,67 |

The above-mentioned results clearly show that the present invention using a multi-part administration regime as defined in the claims is highly effective even in the long-term (or chronic) treatment of severe hyperhidrosis (namely in patients suffering from hyperhidrosis rated as 3 or 4, in particular as 4, in the HDSS).

## Claims

1. A topical formulation comprising at least one anticholinergic compound for use in treating patients suffering from severe hyperhidrosis, wherein administration of the formulation to the patient comprises:
a) an initial phase with a topical administration of a predetermined amount of the formulation 5 to 7 times per week for a total time period of less than six months; followed by
b) a maintenance phase with a topical administration of the same amount of the formulation 1 to less than 5 times per week,
wherein the anticholinergic compound is a glycopyrronium (GP) salt that is contained in an amount of 0.05 to 5 wt.%, based on the weight of the total formulation.

2. The topical formulation for use according to claim 1 in the form of an emulsion, preferably an oil-in-water emulsion comprising
a) a disperse/inner oil phase;
b) a continuous/outer water phase containing the anticholinergic compound; and
c) an emulsifier.

3. The topical formulation for use according to claim 1 and/or claim 2, wherein the anticholinergic compound is selected from the group consisting of the bromide, the acetate, and the tosylate salts of glycopyrronium (GP).

4. The topical formulation for use according to claim 3, wherein the GP salt is a racemic mixture of (3*R*)-3-[(2*S*)-(2-cyclopentyl-2-hydroxy-2-phenylacetyl)oxy]-1,1-dimethylpyrrolidinium bromide and (3*S*)-3-[(2*R*)-(2-cyclopentyl-2-hydroxy-2-phenylacetyl)oxy]-1,1-dimethylpyrrolidinium bromide or a racemic mixture of (3*R*)-3-[(2*S*)-(2-cyclopentyl-2-hydroxy-2-phenylacetyl)oxy]-1,1-dimethylpyrrolidinium tosylate and (3*S*)-3-[(2*R*)-(2-cyclopentyl-2-hydroxy-2-phenylacetyl)oxy]-1,1-dimethylpyrrolidinium tosylate.

5. The topical formulation for use according to claims 3 and/or 4, wherein the GP salt is contained in an amount of 0.1 to 3 wt.%, preferably of 0.2 to 2 wt.%, and more preferably of 0.5 to 1.5 wt.%, based on the weight of the total formulation.

6. The topical formulation for use according to any one of claims 1 to 5, wherein administration of the formulation to the patient comprises:
a) an initial phase with a topical administration of a predetermined amount of the formulation once-daily for a total time period of from two weeks to four months, preferably for a total time period of from three weeks to two months, most preferably four weeks; followed by
b) a maintenance phase with a topical administration of the same amount of the formulation two to four times, preferably two to three times, most preferably two times per week.

7. The topical formulation for use according to any one of claims 1 to 6, wherein the amount of the formulation per administration is within a range of from 200 to 800 mg, preferably from 400 to 600 mg, per application site.

8. The topical formulation for use according to any one of claims 1 to 7, wherein the hyperhidrosis to be treated is selected from the group consisting of primary and secondary hyperhidrosis, gustatory sweating associated with Frey's syndrome, gustatory sweating associated with diabetic autonomic neuropathy, and excessive sweating, preferably axillary hyperhidrosis.

9. Pharmaceutical composition comprising the topical formulation for use according to any one of claims 1 to 8 and one or more pharmaceutically acceptable excipients.

## Patentansprüche

1. Topische Formulierung mit mindestens einer anticholinergen Verbindung zur Verwendung bei der Behandlung von Patienten, die unter schwerer Hyperhidrose leiden, wobei die Verabreichung der Formulierung an den Patienten umfasst:
a) eine anfängliche Phase mit einer topischen Verabreichung einer vorgegebenen Menge der Formulierung 5 bis 7 mal pro Woche für eine gesamte Zeitdauer von weniger als sechs Monaten; gefolgt von
b) einer Aufrechterhaltungsphase mit einer topischen Verabreichung derselben Menge der Formulierung 1 bis weniger als 5 mal pro Woche,
wobei die anticholinerge Verbindung ein Glycopyrroniumsalz (GP-Salz) ist, das in einer Menge von 0,05 bis 5 Gew.-% auf der Basis des Gewichts der gesamten Formulierung enthalten ist.

2. Topische Formulierung zur Verwendung nach Anspruch 1 in Form einer Emulsion, vorzugsweise einer Öl-in-Wasser-Emulsion, die umfasst:
a) eine disperse/innere Ölphase;
b) eine kontinuierliche/äußere Wasserphase, die die anticholinerge Verbindung enthält; und
c) einen Emulgator.

3. Topische Formulierung zur Verwendung nach Anspruch 1 und/oder Anspruch 2, wobei die anticholinerge Verbindung aus der Gruppe ausgewählt ist, die aus den Bromid-, den Acetat- und den Tosylatsalzen von Glycopyrronium (GP) besteht.

4. Topische Formulierung zur Verwendung nach Anspruch 3, wobei das GP-Salz ein racemisches Gemisch von (3*R*)-3-[(2*S*)-(2-Cyclopentyl-2-hydroxy-2-phenylacetyl)oxy]-1,1-dimethylpyrrolidiniumbromid und (3*S*)-3-[(2*R*)-(2-Cyclopentyl-2-hydroxy-2-phenylacetyl)oxy]-1,1-dimethylpyrrolidiniumbromid oder ein racemisches Gemisch von (3*R*)-3-[(2*S*)-(2-Cyclopentyl-2-hydroxy-2-phenylacetyl)oxy]-1,1-dimethylpyrrolidiniumtosylat und (3*S*)-3-[(2*R*)-(2-Cyclopentyl-2-hydroxy-2-phenylacetyl)oxy]-1,1-dimethylpyrrolidiniumtosylat ist.

5. Topische Formulierung zur Verwendung nach den Ansprüchen 3 und/oder 4, wobei das GP-Salz in einer Menge von 0,1 bis 3 Gew.-%, vorzugsweise von 0,2 bis 2 Gew.-%, und bevorzugter von 0,5 bis 1,5 Gew.-% auf der Basis des Gewichts der gesamten Formulierung enthalten ist.

6. Topische Formulierung zur Verwendung nach einem der Ansprüche 1 bis 5, wobei die Verabreichung der Formulierung an den Patienten umfasst:
a) eine anfängliche Phase mit einer topischen Verabreichung einer vorgegebenen Menge der Formulierung einmal täglich für eine gesamte Zeitdauer von zwei Wochen bis vier Monaten, vorzugsweise für eine gesamte Zeitdauer von drei Wochen bis zwei Monaten, am meisten bevorzugt vier Wochen; gefolgt von
b) einer Aufrechterhaltungsphase mit einer topischen Verabreichung derselben Menge der Formulierung zwei- bis viermal, vorzugsweise zwei- bis dreimal, am stärksten bevorzugt zweimal pro Woche.

7. Topische Formulierung zur Verwendung nach einem der Ansprüche 1 bis 6, wobei die Menge der Formulierung pro Verabreichung innerhalb eines Bereichs von 200 bis 800 mg, vorzugsweise von 400 bis 600 mg, pro Anwendungsstelle liegt.

8. Topische Formulierung zur Verwendung nach einem der Ansprüche 1 bis 7, wobei die zu behandelnde Hyperhidrose aus der Gruppe ausgewählt ist, die aus primärer und sekundärer Hyperhidrose, gustatorischem Schwitzen, das mit dem Frey-Syndrom verbunden ist, gustatorischem Schwitzen, das mit diabetischer autonomer Neuropathie verbunden ist, und übermäßigem Schwitzen, vorzugsweise axillärer Hyperhidrose, besteht.

9. Pharmazeutische Zusammensetzung mit der topischen Formulierung zur Verwendung nach einem der Ansprüche 1 bis 8 und einem oder mehreren pharmazeutisch verträglichen Trägerstoffen.

## Revendications

1. Formulation topique comportant au moins un composé anticholinergique pour une utilisation dans le traitement de patients souffrant d'hyperhidrose sévère, dans laquelle l'administration de la formulation au patient comporte :
a) une phase initiale avec une administration topique d'une quantité prédéterminée de la formulation 5 à 7 fois par semaine pendant une période de temps totale inférieure à six mois ; suivie de
b) une phase d'entretien avec une administration topique de la même quantité de la formulation 1 à moins de 5 fois par semaine,
dans laquelle le composé anticholinergique est un sel de glycopyrronium (GP) qui est contenu en une quantité de 0,05 à 5 % en poids, sur la base du poids de la formulation totale.

2. Formulation topique pour une utilisation selon la revendication 1 sous la forme d'une émulsion, de préférence d'une émulsion huile dans eau comportant :
a) une phase huileuse dispersée/interne ;
b) une phase aqueuse continue/externe contenant le composé anticholinergique ; et
c) un émulsifiant.

3. Formulation topique pour une utilisation selon la revendication 1 et/ou la revendication 2, dans laquelle le composé anticholinergique est choisi parmi le groupe constitué du bromure, de l'acétate et des sels de tosylate de glycopyrronium (GP).

4. Formulation topique pour une utilisation selon la revendication 3, dans laquelle le sel de GP est un mélange racémique de bromure de (3*R*)-3-[(2*S*)-(2-cyclopentyl-2-hydroxy-2-phénylacétyl)oxy]-1,1-diméthylpyrrolidinium et de bromure de (3*S*)-3-[(2*R*)-(2-cyclopentyl-2-hydroxy-2-phénylacétyl)oxy]-1,1-diméthylpyrrolidinium, ou d'un mélange racémique de (3*R*)-3-[(2*S*)-(2-cyclopentyl-2-hydroxy-2-phénylacétyl)oxy]-1,1-diméthylpyrrolidinium tosylate et de (3*S*)-3-[(2*R*)-(2-cyclopentyl-2-hydroxy-2-phénylacétyl)oxy]-1,1-diméthylpyrrolidinium tosylate.

5. Formulation topique pour une utilisation selon les revendications 3 et/ou 4, dans laquelle le sel de GP est contenu en une quantité de 0,1 à 3 % en poids, de manière préférée de 0,2 à 2 % en poids, et de manière plus préférée de 0,5 à 1,5 % en poids, sur la base du poids de la formulation totale.

6. Formulation topique pour une utilisation selon l'une quelconque des revendications 1 à 5, dans laquelle l'administration de la formulation au patient comporte :
a) une phase initiale avec une administration topique d'une quantité prédéterminée de la formulation une fois par jour pendant une période de temps totale de deux semaines à quatre mois, de manière préférée pendant une période de temps totale de trois semaines à deux mois, de manière la plus préférée de quatre semaines ; suivie de
b) une phase d'entretien avec une administration topique de la même quantité de formulation deux à quatre fois, de manière préférée deux à trois fois, de manière la plus préférée deux fois par semaine.

7. Formulation topique pour une utilisation selon l'une quelconque des revendications 1 à 6, dans laquelle la quantité de la formulation par administration est dans un intervalle de 200 à 800 mg, de manière préférée de 400 à 600 mg, par site d'application.

8. Formulation topique pour une utilisation selon l'une quelconque des revendications 1 à 7, dans laquelle l'hyperhidrose à traiter est choisie parmi le groupe constitué d'une hyperhidrose primaire et d'une hyperhidrose secondaire, d'une sudation gustative associée au syndrome de Frey, d'une sudation gustative associée à une neuropathie autonome diabétique et d'une sudation excessive, de préférence une hyperhidrose axillaire.

9. Composition pharmaceutique comportant la formulation topique pour une utilisation selon l'une quelconque des revendications 1 à 8 et un ou plusieurs excipients acceptables du point de vue pharmaceutique.
